# EUROPEAN PATENT APPLICATION

(11) **EP 3 680 324 A1**
(43) Date of publication of application: **15.07.2020**
(21) Application number: 18852821.0
(22) Date of filing: 06.09.2018
(51) Int. Cl.: C12N 5/0775

(54) **STEM CELLS DERIVED FROM YOUNG PIG AND PREPARATION METHOD THEREFOR**

(30) Priority: 08.09.2017 US 201762555913 P
(71) Applicant: Otsuka Pharmaceutical Factory, Inc., Tokushima 772-8601 (JP)
(72) Inventor: NISHIMURA Masuhiro, Naruto-shi Tokushima 772-8601 (JP); FUJITA Yasutaka, Naruto-shi Tokushima 772-8601 (JP); WATANABE Natsuki, Naruto-shi Tokushima 772-8601 (JP); NGUYEN Luan, Naruto-shi Tokushima 772-8601 (JP); MATSUMOTO Shinichi, Naruto-shi Tokushima 772-8601 (JP)
(74) Representative: Goulard, Sophie
(86) International application number: PCT/JP2018/033110
(87) International publication number: WO 2019/049957

(57) **Abstract**

An object of the present invention is to provide stem cells having excellent proliferation ability and differentiation ability. The present invention relates to stem cells isolated from a neonatal pig, and a preparation method therefor.

## Description

### Technical Field

The present invention relates to neonatal pig-derived stem cells and a preparation method therefor, more particularly relates to mesenchymal stem cells derived from a neonatal pig that can differentiate into adipocytes, osteocytes, and chondrocytes and a preparation method therefor.

### Background Art

Due to the recent advance of research in somatic stem cells including mesenchymal stem cells, clinical application of somatic stem cells has already been shifted from basic research stage to development stage. Somatic stem cells have three major functions (pluripotency, immunoregulatory ability, and remodeling ability in the extracellular environment), and are expected as cells for treating a refractory disease.

Firstly, the pluripotency is an ability of somatic stem cells to directly differentiate into bone, cartilage, or the like, and administered somatic stem cells complement lost cells or substitute for cells having an insufficient function, thereby exhibiting a therapeutic effect.

Secondly, the immunoregulatory ability works on immunocompetent cells of a patient through secretion of an anti-inflammatory cytokine, chemokine, exosome, or the like from somatic stem cells or through an intercellular adhesion molecule or the like so as to suppress an inflammation or an immunoreaction in a graft-versus-host disease or the like, thereby exhibiting a therapeutic effect.

Thirdly, with respect to the remodeling ability in the extracellular environment, a therapeutic effect is exhibited by secretion of a vascular inducing factor, a growth factor, an antifibrotic factor, or the like from somatic stem cells in an infarct site in an ischemic disease, a fibrotic site caused by an inflammation, or the like.

Mesenchymal stem cells are somatic stem cells present in the bone marrow, fat, pancreatic islet, umbilical cord blood, and the like of a mammal, and derived from a mesodermal tissue (mesenchyme) and have an ability to differentiate into cells belonging to the mesenchymal lineage. Recently, a clinical trial has been carried out for diseases such as a graft-versus-host disease, a cardiovascular disorder, an autoimmune disease, osteoarthritis, dysostosis, a hepatic disorder, a respiratory disease, spinal cord injury, cerebral infarction, and renal failure (Non-Patent Document 1).

Mesenchymal stem cells have been expected to be used in various clinical applications, but have problems with respect to securing of a donor, invasion in a donor, security of safety such as a virus free test for each donor, and the like. Further, the effect of mesenchymal stem cells to be obtained varies largely depending on the donor or the conditions such as the age of the donor, and securing of stable quality of cells for treatment is also a big problem. A technique of proliferating mesenchymal stem cells derived from the bone marrow or the like of a patient in vitro and using the cells for treating the same patient can become an alternative treatment method for tissue/organ transplantation that has been at issue due to the lack of donors. However, there is an individual difference in the proliferation ability and differentiation ability of cells, and cells derived from all patients do not exhibit the same behavior (Non-Patent Document 2). As described above, securing of a donor, confirmation of safety, and excellent proliferation and differentiation abilities of stem cells are required for preparing sufficient stem cells for treatment.

### Citation List

### Non-Patent Documents

Non-Patent Document 1: Lemos NE, de Almeida Brondani L, Dieter C, Rheinheimer J, Boucas AP, Bauermann Leitao C, Crispim D, Bauer AC. Islets. 2017 Jul 5: e1335842. doi: 10.1080/19382014. 2017. 1335842

Non-Patent Document 2: Hajime Ohgushi, Biochemistry, Vol. 81, No. 2, Pages 99 to 104, February 2009

### Summary of Invention

### Problems to Be Solved by the Invention

As described above, in the case where stem cells are used in regenerative therapy, securing of a donor, confirmation of safety, and excellent proliferation and differentiation abilities of stem cells are required. However, after multiple subcultures of stem cells, their proliferation ability and differentiation ability are decreased, and there is a problem that it takes cost and time for preparing sufficient stem cells for treatment. Therefore, an object of the present invention is to provide stem cells having excellent proliferation ability and differentiation ability using a neonatal medical pig which enables stable supply or management of pathogens as a donor source.

### Means for Solving the Problems

The present inventors found that mesenchymal stem cells prepared from the bone marrow of a neonatal pig have excellent properties as follows: a significantly high growth rate, an excellent proliferation ability, and a small cell size as compared with conventional mesenchymal stem cells, and thus completed the present invention.

That is, the present invention relates to the following.
1. Stem cells isolated from a neonatal pig.
2. The stem cells according to above 1, wherein an average diameter is 17 µm or less.
3. The stem cells according to above 1 or 2, wherein a doubling time in a logarithmic growth phase is 36 hours or less.
4. The stem cells according to any one of above 1 to 3, wherein the stem cells are mesenchymal stem cells.
5. The stem cells according to any one of above 1 to 4, wherein the neonatal pig is a neonatal pig that enables cell transplantation into a human.
6. The stem cells according to any one of above 1 to 5, wherein the stem cells are isolated from the bone marrow or the pancreatic islet of the neonatal pig.
7. The stem cells according to any one of above 1 to 6, wherein the stem cells are stem cells for transplantation.
8. The stem cells according to above 7, wherein the stem cells are stem cells for human transplantation.
9. The stem cells according to any one of above 1 to 6, wherein the stem cells are stem cells for feeder cells.
10. The stem cells according to above 9, wherein the stem cells are stem cells for feeder cells for proliferating stem cells for human transplantation.
11. A method for preparing stem cells, comprising a step of isolating cells from a neonatal pig.
12. The method for preparing stem cells according to above 11, comprising a step of subculturing the stem cells after 3 to 12 days from inoculation.
13. The method for preparing stem cells according to above 11 or 12, wherein the cells to be isolated from the neonatal pig are cells in a monocytic cell fraction.
14. The method for preparing stem cells according to any one of above 11 to 13, wherein the isolation of the cells from the neonatal pig is isolation of the cells from the bone marrow or the pancreatic islet of the neonatal pig.
15. The method for preparing stem cells according to above 13 or 14, comprising a step of freezing the isolated cells in the monocytic cell fraction.

### Effects of the Invention

The stem cells of the present invention have advantages as follows: a significantly high growth rate, an excellent proliferation ability, and a small cell size as compared with conventional stem cells. Since the stem cells of the present invention have a significantly high growth rate, a large amount of stem cells to be used for applications such as for transplantation or for feeder cells can be acquired in a short time and at low cost. Further, by administration of stem cells, the lung is clogged with the stem cells to cause pulmonary embolism in some cases, however, since the stem cells of the present invention have a small cell size, such formation of pulmonary embolism can be prevented.

### Brief Description of Drawings

[Fig. 1] Fig. 1A is a view showing a total cell amount for a specific culture period (days) when stem cells of the present invention were cultured. Fig. 1B is a view showing a total cell growth rate for a specific culture period (days) when stem cells of the present invention were cultured. In Fig. 1A and Fig. 1B, a dotted line and black circles show neonatal porcine bone marrow-derived mesenchymal stem cells (npBM-MSC), and a solid line and white circles show human bone marrow-derived mesenchymal stem cells (hBM-MSC).
[Fig. 2] Fig. 2A and Fig. 2B are views showing differentiation into adipocytes with respect to human bone marrow-derived mesenchymal stem cells (hBM-MSC) and neonatal porcine bone marrow-derived mesenchymal stem cells (npBM-MSC), respectively.
[Fig. 3] Fig. 3A and Fig. 3B are views showing differentiation into osteocytes with respect to human bone marrow-derived mesenchymal stem cells (hBM-MSC) and neonatal porcine bone marrow-derived mesenchymal stem cells (npBM-MSC), respectively.
[Fig. 4] Fig. 4A and Fig. 4B are views showing differentiation into osteocytes with respect to human bone marrow-derived mesenchymal stem cells (hBM-MSC) and neonatal porcine bone marrow-derived mesenchymal stem cells (npBM-MSC), respectively.
[Fig. 5] Fig. 5A and Fig. 5B are views showing differentiation into chondrocytes with respect to neonatal porcine bone marrow-derived mesenchymal stem cells (npBM-MSC).
[Fig. 6] Fig. 6 is a view showing results of a cell surface antigen analysis of neonatal porcine bone marrow-derived mesenchymal stem cells (npBM-MSC) using CD44 that is a marker for mesenchymal stem cells.
[Fig. 7] Fig. 7 is a view showing results of a cell surface antigen analysis of neonatal porcine bone marrow-derived mesenchymal stem cells (npBM-MSC) using CD90 that is a marker for mesenchymal stem cells.

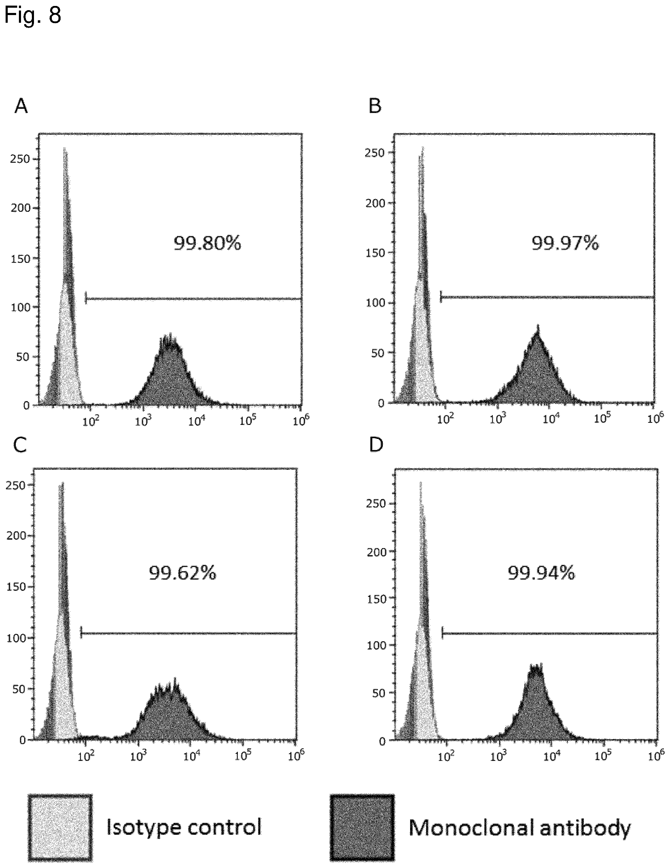

[Fig. 8] Fig. 8A to Fig. 8D are views showing results of a cell surface antigen analysis of neonatal porcine pancreatic islet-derived mesenchymal stem cells (npISLET-MSC) using CD29 that is a marker for mesenchymal stem cells. Fig. 8A shows the results of Sample 11 (without freezing immediately after preparing the pancreatic islet), Fig. 8B shows the results of Sample 12 (with freezing immediately after preparing the pancreatic islet), Fig. 8C shows the results of Sample 13 (without freezing on day 3 of culture after preparing the pancreatic islet), and Fig. 8D shows the results of Sample 14 (with freezing on day 3 of culture after preparing the pancreatic islet).

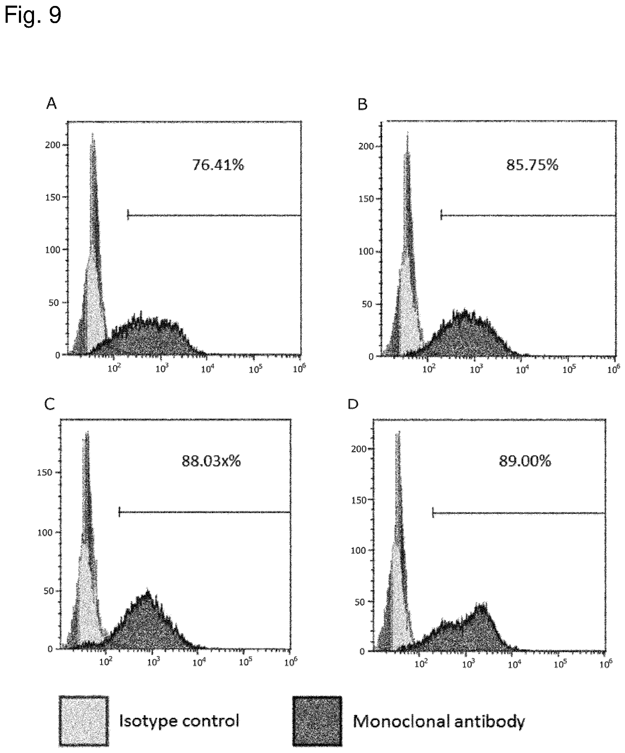

[Fig. 9] Fig. 9A to Fig. 9D are views showing results of a cell surface antigen analysis of neonatal porcine pancreatic islet-derived mesenchymal stem cells (npISLET-MSC) using CD44 that is a marker for mesenchymal stem cells. Fig. 9A shows the results of Sample 11 (without freezing immediately after preparing the pancreatic islet), Fig. 9B shows the results of Sample 12 (with freezing immediately after preparing the pancreatic islet), Fig. 9C shows the results of Sample 13 (without freezing on day 3 of culture after preparing the pancreatic islet), and Fig. 9D shows the results of Sample 14 (with freezing on day 3 of culture after preparing the pancreatic islet).

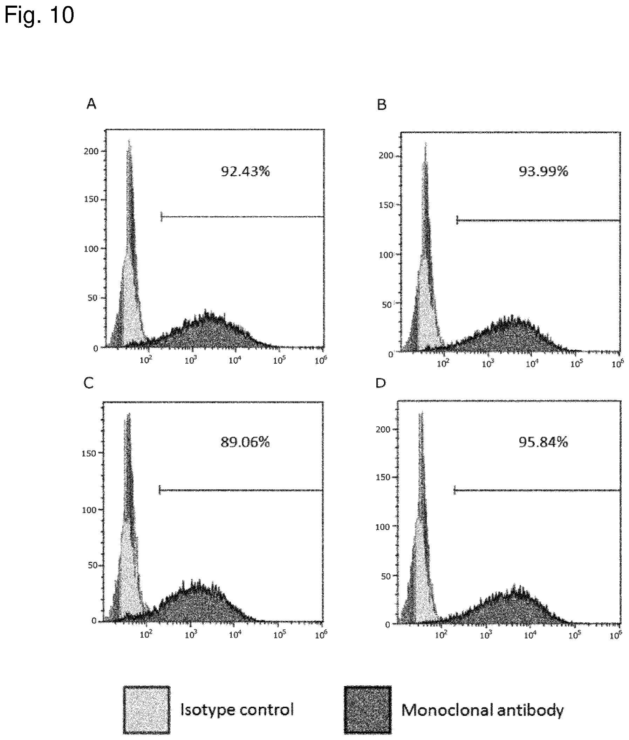

[Fig. 10] Fig. 10A to Fig. 10D are views showing results of a cell surface antigen analysis of neonatal porcine pancreatic islet-derived mesenchymal stem cells (npISLET-MSC) using CD90 that is a marker for mesenchymal stem cells. Fig. 10A shows the results of Sample 11 (without freezing immediately after preparing the pancreatic islet), Fig. 10B shows the results of Sample 12 (with freezing immediately after preparing the pancreatic islet), Fig. 10C shows the results of Sample 13 (without freezing on day 3 of culture after preparing the pancreatic islet), and Fig. 10D shows the results of Sample 14 (with freezing on day 3 of culture after preparing the pancreatic islet).

### Embodiments for Carrying Out the Invention

The stem cells of the present invention are stem cells isolated from a neonatal pig. Incidentally, the stem cells described in the below-mentioned Examples were separated from the bone marrow or the pancreatic islet of a neonatal pig. However, for example, stem cells derived from the skin, fat, or the like of a neonatal pig are also included in the present invention.

In the present invention, the "neonatal pig" refers to a fetal pig or a pig less than one month after birth, preferably less than 25 days after birth. The neonatal pig is preferably a pig for medical use, and more preferably a neonatal pig that enables cell transplantation into a human. The breed of the pig is not particularly limited, however, examples thereof include Landrace breed (for example, Danish Landrace breed, American Landrace breed, British Landrace breed, Dutch Landrace breed, and Swedish Landrace breed), Large White Yorkshire breed, Berkshire breed, Duroc breed, Hampshire breed, Middle White Yorkshire breed, and a miniature pig, and above all, Landrace breed is preferred.

In general, the "stem cells" mean immature cells having a self-replication ability and differentiation and proliferation abilities. In the stem cells, according to the differentiation ability, subpopulations such as pluripotent stem cells, multipotent stem cells, and unipotent stem cells are included.

The pluripotent stem cells mean cells that cannot become an individual by themselves, but have an ability to be able to differentiate into all tissues or cells constituting a living body. The multipotent stem cells mean cells that have an ability to be able to differentiate into not all types, but a plurality of types of tissues or cells. The unipotent stem cells mean cells that have an ability to be able to differentiate into a specific tissue or cell.

As the stem cells of the present invention, multipotent stem cells are preferred. Examples of the multipotent stem cells include somatic stem cells such as mesenchymal stem cells, hematopoietic stem cells, neural stem cells, bone marrow stem cells, and germline stem cells, and mesenchymal stem cells are preferred.

As long as the stem cells of the present invention are stem cells isolated from a neonatal pig, stem cells that are primary cultured cells thereof and cells obtained by subculturing the primary cultured cells, and can generate various types of cells expressing various types of differentiation markers are also included in the stem cells of the present invention. In addition, when the stem cells of the present invention are mesenchymal stem cells, it is preferred that 60% or more of the cells are positive for CD44 and CD90 that are cell markers, more preferably 70% or more, and further more preferably 80% or more of the cells are positive for CD44 and CD90. Further, it is preferred that 60% or more of the cells are positive for CD29 that is a cell marker, more preferably 70% or more, and further more preferably 80% or more of the cells are positive for CD29.

In the stem cells of the present invention, a doubling time in a logarithmic growth phase is preferably 36 hours or less, more preferably 32 hours or less, further more preferably 28 hours or less, particularly preferably 24 hours or less, and most preferably 20 hours or less. In addition, the doubling time in a logarithmic growth phase is preferably 14 hours or more, and more preferably 16 hours or more.

The culture in a logarithmic growth phase of the stem cells of the present invention can be performed by inoculating the stem cells of the present invention into a medium containing vitamin C described below (for example, MSC medium) and culturing the cells in an incubator for culture at 37°C in the presence of 5% CO₂. As the doubling time in the logarithmic growth phase is shorter, it becomes possible to prepare a large amount of stem cells in a short time and at low cost.

The stem cells of the present invention have an average diameter of preferably 17 µm or less, more preferably 16.5 µm or less, further more preferably 16 µm or less, particularly preferably 15.5 µm or less, and most preferably 15 µm or less. The average diameter is preferably 10 µm or more, more preferably 12 µm or more. As the average diameter is smaller, formation of pulmonary embolism by administration of the stem cells can be prevented. The average diameter can be measured using, for example, Nucleo Counter NC-200 (trademark). Here, the average refers to arithmetic average.

In the differentiation from the mesenchymal stem cells of the present invention into adipocytes, for example, by culturing the mesenchymal stem cells of the present invention in the presence of insulin, MCGS (a serum component, Mesenchymal Stem Cell Growth Supplement), dexamethasone, indomethacin, isobutyl methylxanthine, and the like, the differentiation into adipocytes can be induced.

In the differentiation into adipocytes and the maintenance, a commercially available kit or medium, or the like may be used, and examples thereof include hMSC differentiation BulletKit (trademark)-adipogeni (PT-3004) manufactured by Lonza Walkersville, Inc., hMSC adipogenic induction medium (PT-3102B) manufactured by Lonza Walkersville, Inc., hMSC adipogenic maintenance medium (PT-3102B) manufactured by Lonza Walkersville, Inc., and the like. The differentiation from mesenchymal stem cells into adipocytes can be confirmed using a commercially available kit, and examples thereof include Adipo Red (trademark) assay reagent manufactured by Lonza, Inc.

In the differentiation from the mesenchymal stem cells of the present invention into osteocytes, for example, by culturing the mesenchymal stem cells of the present invention in the presence of dexamethasone, an ascorbate salt, MCGS, β-glycerophosphoric acid, and the like, the differentiation into osteocytes can be induced. In addition, a commercially available kit may be used, and examples thereof include hMSC differentiation BulletKit (trademark)-osteogenic, PT-3004 manufactured by Lonza Walkersville, Inc., and the like. The differentiation from mesenchymal stem cells into osteocytes can be confirmed using a commercially available alkaline phosphatase staining kit (for example, manufactured by Cosmo Bio Co., Ltd., or the like), a commercially available calcification staining kit (for example, manufactured by Cosmo Bio Co., Ltd., or the like), or the like.

In the differentiation from the mesenchymal stem cells of the present invention into chondrocytes, for example, by culturing the mesenchymal stem cells of the present invention in the presence of TGF-β3, dexamethasone, insulin-transferrin-selenious acid (ITS), sodium pyruvate, proline, and an ascorbate salt, the differentiation into chondrocytes can be induced. In addition, a commercially available kit may be used, and examples thereof include hMSC differentiation BulletKit (trademark)-condrogenic, PT-3003 manufactured by Lonza Walkersville, Inc., and the like. The differentiation from mesenchymal stem cells into chondrocytes can be confirmed by Alcian blue staining, or the like.

The transplantation of the stem cells can be easily performed by injecting a suspension of the stem cells into a host body. The injection can be performed into an organ to be treated by regenerative therapy or the vicinity thereof or into the vein or the like. Further, the number of stem cells to be injected is not particularly limited, and can be appropriately selected according to the symptoms, the body weight of the host, or an administration method, or the like, but is generally set to about 10² to 10¹⁰.

In the present description, the "feeder cells" refer to another cell type that plays an assisting role and is used for adjusting the culture conditions for target cells to be proliferated or differentiated.

When cells are used as the feeder cells, the cells have been irradiated with a gamma ray or treated with an antibiotic in advance so as to prevent regular proliferation. As the feeder cells for the stem cells, fibroblasts derived from a mouse embryo are mainly used, and various cell types such as fibroblasts including 3T3, SNL, and the like are used as the feeder cells depending on the purpose of an experiment or the cells. The stem cells of the present invention can be preferably used as the feeder cells for stem cells for human transplantation by isolating the stem cells from a neonatal pig that enables cell transplantation into a human.

A method for preparing stem cells of the present invention is characterized by including a step of isolating cells from a neonatal pig. One embodiment of the method for preparing stem cells of the present invention is, for example, the method comprising the following steps.
(1) a step of collecting cells from a neonatal pig
(2) a step of preparing neonatal pig-derived stem cells by culturing the cells collected in the step (1)

Hereinafter, the respective steps will be described.

### (1) Step of collecting cells from neonatal pig

In the step (1), cells are collected from the bone marrow, fat, skin, pancreas, or the like of a neonatal pig.

Specifically, for example, when cells are collected from the bone marrow of a neonatal pig, bone marrow cells can be collected from the femur, iliac crest, breast bone, or the like of a neonatal pig. For example, the femur is collected from a neonatal pig, both ends are cut off, a needle is inserted, washing off is performed with a physiological buffer solution (for example, a phosphate buffer solution, hereinafter also referred to as PBS) supplemented with heparin, and an outflow liquid from a place on the opposite side is recovered as a bone marrow liquid. When the amount of the outflow liquid is decreased, the bone is reversed and the needle is inserted on the opposite side, and then washing off is performed again with PBS and a bone marrow liquid that is a cell-containing solution is prepared.

Further, a neonatal pig-derived monocytic cell fraction may be isolated by conventional centrifugation of the cell-containing solution prepared above. The cell-containing solution prepared above is diluted with PBS or the like, and in a tube containing a medium for separation of human lymphocytes (for example, Ficoll-Paque PLUS manufactured by GE Healthcare Life Sciences, or the like), the diluted cell-containing solution is placed on a layer of the medium.

The tube is centrifuged to separate layers, and a layer containing neonatal pig-derived monocytic cells is recovered. The recovered solution is further centrifuged, and the supernatant is removed. Then, the resultant is diluted with PBS or the like, followed by centrifugation again, whereby a monocytic cell fraction is isolated. The cells in the monocytic cell fraction isolated in this manner may be cryopreserved before culture. By freezing the cells in the isolated neonatal pig-derived monocytic cell fraction, cells that are less likely to be affected by freezing and thawing can be selectively prepared. When the cells are cryopreserved before culture, the temperature is preferably -80°C or lower, and more preferably -150°C or lower.

Further, for example, when cells are collected from the pancreas of a neonatal pig, the pancreatic islet is collected from a neonatal pig. Furthermore, in some cases, the pancreatic islet is subjected to suspension culture, whereby a cell cluster to be used in adhesion culture for the purpose of preparing stem cells is prepared.

Further, for example, when cells are collected from the fat of a neonatal pig, the fat is collected from a neonatal pig and cut into fine pieces with scissors, followed by an enzyme treatment. The resultant is filtered through a cell strainer, and then centrifuged at a low speed. The cells precipitated on the bottom of the tube are used for culture. In addition, for example, when cells are collected from the skin (including hair) of a neonatal pig, the skin is collected from a neonatal pig and is subjected to an enzyme treatment. After the enzyme treatment, the hair is removed from the skin, and a bulge portion is collected and used for culture. When culture is performed, 3T3 feeder cells are used.

### (2) Step of preparing neonatal pig-derived stem cells by culturing cells collected in step (1)

In the cells, the cell fraction, or the cell cluster collected in the above step (1), unintended cells other than stem cells are contained in a large amount. In general, a culture method in which such cells are removed by using a minimal essential medium not containing vitamin C, which is essential for survival of such unintended cells (for example, the below-mentioned MSC minimal essential medium) is used.

In the step (2) of the present invention, the cells, the cell fraction, or the cell cluster collected in the above step (1) are/is cultured in an incubator for culture preferably at 35 to 39°C, more preferably at 36 to 38°C, and most preferably at 37°C in the presence of CO₂ preferably at 4 to 6%, more preferably at 4.5 to 5.5%, and most preferably at 5%, whereby the unintended cells which are other than stem cells are removed, and also the stem cells of the present invention are proliferated.

The stem cells of the present invention have a significantly high growth rate, and therefore, the stem cells of the present invention can be prepared by using only a medium containing vitamin C (for example, the below-mentioned MSC medium), instead of using a minimal essential medium not containing vitamin C, for culture to remove the above-mentioned unintended cells. Incidentally, the stem cells of the present invention can also be prepared by culture using a minimal essential medium not containing vitamin C for removing the above-mentioned unintended cells, and thereafter replacing the medium with a medium containing vitamin C so as to proliferate the stem cells of the present invention.

The stem cells of the present invention are cultured by, specifically, for example, the following method. A container for culture coated with gelatin (for example, a plate coated with 0.1% gelatin) or a container for culture without gelatin coat (for example, a plate) is used, and a minimal essential medium not containing vitamin C (for example, the below-mentioned MSC minimal essential medium) or a medium containing vitamin C (for example, the below-mentioned MSC medium) is used, and primary cultured cells are obtained by inoculating the cells preferably at 5.0×10⁵ cells to 5.0×10⁷ cells/9.6 cm², and incubating the cells, for example, under the conditions of 37°C, 5% CO₂, and 90% humidity. The culture period for obtaining the primary cultured cells is preferably 3 to 12 days, more preferably 3 to 11 days, and most preferably 3 to 10 days after inoculation. The primary cultured cells may be subcultured. The stem cells obtained by subculture are also referred to as subcultured cells. The subculture of the primary cultured cells or the subcultured cells is performed after the stem cells reached 30% to 100% confluence, preferably 50% to 95% confluence, more preferably 60% to 90% confluence, and most preferably 70% to 85% confluence preferably after 2 to 6 days, more preferably after 2 to 5 days, further more preferably after 2 to 4 days, and most preferably after 3 days from the inoculation of the stem cells. In the inoculation of the stem cells, the cells are inoculated preferably at 5.0×10⁵ cells to 5.0×10⁷ cells/9.6 cm² using a container for culture coated with gelatin (for example, a plate coated with 0.1% gelatin) or a container for culture without gelatin coat (for example, a plate), and using a medium containing vitamin C (for example, the below-mentioned MSC medium). In the culture of the stem cells, the cells are cultured, for example, under the conditions of 37°C, 5% CO₂, and 90% humidity. The stem cells of the present invention are proliferated by replacing a medium as needed during the culture of the stem cells.

As the MSC minimal essential medium and the MSC medium, conventionally known media can be used, and commercially available media may be used. Examples of the MSC minimal essential medium include a medium obtained by adding 55 mL of fetal bovine serum (FBS) manufactured by Gibco, Inc. and 5.5 mL of penicillin-streptomycin manufactured by Sigma-Aldorich Co. LLC to 500 mL of MEMα (nucleosides, no ascorbic acid) manufactured by Gibco, Inc. Further, examples of the MSC medium include a medium obtained by adding 55 mL of fetal bovine serum (FBS) manufactured by Gibco, Inc., 5.5 mL of penicillin-streptomycin manufactured by Sigma-Aldorich Co. LLC, and 22.2 µL of FGF-Basic, recombinant, expressed in E.coli, suitable for cell culture (final concentration: 1 ng/mL) manufactured by Sigma-Aldorich Co. LLC to 500 mL of MEMα (nucleosides) manufactured by Gibco, Inc.

The subculture is preferably performed at least one or more times. The number of subcultures is not particularly limited as long as the stem cells of the present invention are obtained, but is preferably 1 to 3, more preferably 1 to 20.

The stem cells of the present invention can be cryopreserved. The timing of cryopreservation is not particularly limited, but is preferably after 1 to 20 subcultures, more preferably after 2 to 10 subcultures. As the cryopreserving and thawing methods, conventionally known methods can be used.

As the method for cryopreserving the stem cells, specifically, for example, the cells are dispersed in a cryopreservation solution, and can be cryopreserved at -80°C or lower in a freezer or in liquid nitrogen until they are needed. Examples of the cryopreservation solution include a solution obtained by mixing OPF-301 [a Ringer's lactate solution containing 3% trehalose and 5% dextran (WO 2014/208053)] and dimethyl sulfoxide (DMSO) at a ratio of 9:1, a serum-containing or serum-free preservation solution that can be used for cryopreservation of animal cells, or a commercially available reagent for cell cryopreservation [preferably, a cell banker such as CELLBANKER (registered trademark) manufactured by Takara Bio, Inc.].

### Examples

### Test Example 1

### [Recovery of Neonatal Pig-Derived Bone Marrow Cells]

The bone marrow was collected from the femur of a neonatal pig. The femur was collected from a neonatal pig (a medical Landrace breed pig, 23 days after birth), both ends were cut off, a 12 G needle was inserted, washing off was performed with 50 mL of PBS treated with heparin (3 mL of heparin (1000 U/mL) and 47 mL of PBS), and 50 mL of an outflow liquid of the bone marrow (hereinafter also abbreviated as bone marrow liquid) was recovered from a place on the opposite side. When the amount of the outflow liquid was decreased, the bone was reversed and the needle was inserted on the opposite side, and then washing off was performed again with PBS and a bone marrow liquid was collected. In a 15-mL conical tube for counting, 50 µL of a sample was taken in 1950 µL of PBS (40-fold dilution), and the number of cells was measured using a cell counter.

### [Isolation of Neonatal Pig-Derived Monocytic Cell (npMNC) Fraction]

The bone marrow liquid obtained by the above-mentioned procedure was calmly resuspended. The entire bone marrow liquid was dispensed into four 50-mL tubes in an amount of 10 mL each, and each liquid was diluted to 30 mL with PBS and mixed well while confirming that the cells did not adhere to the tube. To four new 50-mL tubes, 10 mL of Ficoll-Paque PLUS (manufactured by GE Healthcare Life Sciences) was added, and 30 mL of the bone marrow liquid mixed with PBS was placed on the layer of Ficoll-Paque PLUS.

Each of the tubes was centrifuged at 20°C for 30 minutes at 400×g, the speed was slowly accelerated (1/3 the full speed) without applying the break, whereby three different layers were formed. The monocytic cell fraction was positioned in a suspended white ring, and therefore, the entire white ring was collected in a 50-mL tube (×4) containing 25 mL of PBS. Centrifugation was performed at room temperature for 7 minutes at 400×g, and the supernatant was removed. PBS was added up to 40 mL, and centrifugation was performed again at room temperature for 7 minutes at 400×g. When the number of cells was measured in the same manner as described above, 25 to 30% cells were isolated as the monocytic cell fraction ((20 to 30) × 10⁶ cells each) among the entire bone marrow cells.

### [Cryopreservation of Cells in Neonatal Pig-Derived Monocytic Cell (npMNC) Fraction]

The isolated cells in the monocytic cell fraction were placed in a cryovial containing FBS mixed with DMSO (90% FBS and 10% DMSO) at 10⁷ cells/mL, and the total volume of the cell suspension was set to 1 mL [cell number/10×10⁶ = the volume (mL) of FBS mixed with DMSO]. The cryovial was stored at -20°C for 1 hour, and subsequently stored at -80°C for 24 hours, and then finally transferred to a liquid nitrogen tank for long-term storage.

### [Culture of Cells in Neonatal Pig-Derived Monocytic Cell (npMNC) Fraction and Preparation of Neonatal Porcine Bone Marrow-Derived Mesenchymal Stem Cells (npBM-MSC)]

The cell suspension containing the cells in the neonatal pig-derived monocytic cell (npMNC) fraction cryopreserved in the cryovial was promptly thawed in a water bath at 37°C, and the thawed cell suspension was calmly added to 30 mL of MSC minimal essential medium [a medium obtained by adding 55 mL of fetal bovine serum (FBS) manufactured by Gibco, Inc. and 5.5 mL of penicillin-streptomycin manufactured by Sigma-Aldorich Co. LLC to 500 mL of MEMα (nucleosides, no ascorbic acid) manufactured by Gibco, Inc., hereinafter the same shall apply] adjusted to reach the temperature equilibrium (37°C) using a micropipette. The resultant was centrifuged at room temperature for 5 minutes at 500×g, and the resulting pellet was resuspended in 4 mL of the temperature-equilibrated MSC minimal essential medium, and the suspension was gently pipetted up and down. As a result of measurement of the total number of cells and the number of viable cells, the total number of cells was 4.18×10⁶, the number of viable cells was 6.6×10⁵, and the viability was 15.8%.

A 6-well plate was coated with 0.1% gelatin and left to stand for 10 to 15 minutes in an incubator (37°C, 5% CO₂), and thereafter, the gelatin was removed before use. The cell suspension was added to each of the prepared 0.1% gelatin-coated 6-well plate, and the cell suspension was dispersed on a growth surface (gelatin coat) by gently shaking, and the cells were inoculated into 2 mL of the MSC minimal essential medium at 2.09×10⁶ cells/well. In a CO₂ incubator, the cells were cultured under the conditions of 37°C, 5% CO₂, and 90% humidity, and after 3 days, the medium was replaced with MSC medium [a medium obtained by adding 55 mL of fetal bovine serum (FBS) manufactured by Gibco, Inc., 5.5 mL of penicillin-streptomycin manufactured by Sigma-Aldorich Co. LLC, and 22.2 µL of FGF-Basic, recombinant, expressed in E.coli, suitable for cell culture (final concentration: 1 ng/mL) manufactured by Sigma-Aldorich Co. LLC to 500 mL of MEMα (nucleosides) manufactured by Gibco, Inc., hereinafter the same shall apply] so as to proliferate the cells. Thereafter, the MSC medium was replaced with a fresh one every three days. The neonatal porcine bone marrow-derived mesenchymal stem cells (npBM-MSC) reached confluence after 10 days. Also in the case of using a plate without gelatin coat, the neonatal porcine bone marrow-derived mesenchymal stem cells (npBM-MSC) reached confluence after 10 days in the same manner.

### [Subculture]

After the neonatal porcine bone marrow-derived mesenchymal stem cells (npBM-MSC) reached nearly 100% confluence, the cells were recovered from two wells and reinoculated into a T75 flask with or without 0.1% gelatin coat.

The cells were washed with 2 mL of PBS (not containing calcium and magnesium), 320 µL of 0.25% trypsin was added per well, and the cells were left to stand for a few minutes in an incubator. After the cells were peeled off, the medium was neutralized with 1680 µL of the MSC medium. The cell suspension was collected in a 50-mL tube using a 1-mL pipette, and 16 mL (8 mL × 2 wells) of the MSC medium was added thereto, followed by centrifugation at room temperature for 5 minutes at 500×g. The obtained pellet was gently resuspended in the temperature-equilibrated MSC medium (2 mL) using a pipette. As a result of measurement of the total number of cells and the number of viable cells, the total number of cells was 2.05×10⁶, the number of viable cells was 2.02×10⁶, and the viability was 98.5%.

The MSC medium was added to T75 flasks with and without 0.1% gelatin coat, and the cells were reinoculated into the T75 flasks at 45×10⁵ viable cells/flask, and in a CO₂ incubator, the cells were cultured under the conditions of 37°C, 5% CO₂, and 90% humidity. The cells were defined as a first subculture. After 3 days from the inoculation of the first subculture, 100% confluence was reached regardless of the presence or absence of the 0.1% gelatin coat.

### [Preparation of Neonatal Porcine Bone Marrow-Derived Mesenchymal Stem Cells (npBM-MSC)]

After the neonatal porcine bone marrow-derived mesenchymal stem cells (npBM-MSC) reached nearly 100% confluence, the cells were recovered from two flasks of the T75 flasks with or without 0.1% gelatin coat. The cells were washed with 8 mL of PBS (-), 2.4 mL of 0.25% trypsin was added per well, and the cells were left to stand for a few minutes in an incubator. After the cells were peeled off, the medium was neutralized with 12.6 mL of the MSC medium. The cell suspension was collected in a 50-mL tube, followed by centrifugation at room temperature for 5 minutes at 500×g.

To the obtained pellet, the temperature-equilibrated MSC medium (10 mL) was added, and the pellet was calmly resuspended by pipetting up and down, and the results of measurement of the total number of cells and the number of viable cells are shown below.

The cells from the flask (×2) coated with 0.1% gelatin: the total number of cells: 1.62×10⁷, the number of viable cells: 1.60×10⁷, and the viability: 98.8%

The cells from the flask (×2) without gelatin coat: the total number of cells: 1.48×10⁷, the number of viable cells: 1.46×10⁷, and the viability: 98.6%

### [Cryopreservation of Neonatal Porcine Bone Marrow-Derived Mesenchymal Stem Cells (npBM-MSC)]

Apart from the above-mentioned culture, the early subculture neonatal porcine bone marrow-derived mesenchymal stem cells (npBM-MSC) were frozen, whereby a cell stock was prepared. An npBM-MSC pellet that was treated with trypsin in a solution obtained by mixing CELLBANKER (registered trademark) 1 or OPF-301 [a Ringer's lactate solution containing 3% trehalose and 5% dextran (WO 2014/208053)] at a desired concentration and DMSO at a ratio of 9:1 was resuspended to 1.5×10⁶ cells/mL/vial. The vial was placed in a Vi-CELL and stored at -80°C for 24 hours, and thereafter, the cells were transferred to liquid nitrogen from -80°C and stored for a long period of time.

### [CFU Assay]

Into a 21-cm² culture dish (without gelatin coat or with 0.1% gelatin coat), 630 cells of the neonatal porcine bone marrow-derived mesenchymal stem cells (npBM-MSC) (P2) were inoculated at a density of 30 cells/cm², and the cells were cultured in the MSC medium. The MSC medium was replaced with a fresh one every three days. After culture for 6 days, adherent cells were washed twice with 4 mL of PBS, and then fixed with 4 mL of ice-cooled methanol at 4°C for 15 minutes. In order to visualize colonies, the cells were stained for 30 minutes with 4 mL of Giemsa diluted to 1:19 with a phosphate buffer solution, and thereafter washed at room temperature (RT) and washing was performed twice with H₂O.

Subsequently, the number of colonies including more than 50 cells was measured, and the colony formation efficiency of the cells was calculated. The colony formation efficiency of the cells was calculated by dividing the number of colonies per dish by the number of cells (630) inoculated per dish. The results are shown in Table 1. Note that the values in Table 1 each show the average ± SD (n=3).

**[Table 1]**

| Colony formation efficiency of cells (%) | |
|---|---|
| 0.1% gelatin coat | 21.1 ± 3.3 |
| without gelatin coat | 23.1 ± 3.7 |

As shown in Table 1, as a result of the CFU assay, it was found that the obtained neonatal porcine bone marrow-derived mesenchymal stem cells (npBM-MSC) can form colonies regardless of the presence or absence of the gelatin coat.

### [Average Diameter of Cells]

With respect to human bone marrow-derived mesenchymal stem cells (hBM-MSC, the number of subculture: P4) and the obtained neonatal porcine bone marrow-derived mesenchymal stem cells (npBM-MSC), the results of measurement of the average diameter of the cells are shown in Table 2. The average diameter of the cells was measured using Nucleo Counter NC-200 (trademark), and the average value (n=3) was calculated.

**[Table 2]**

| Average Diameter of Cells | |
|---|---|
| npBM-MSC | 13.2 µm |
| hBM-MSC | 17.5 µm |

As shown in Table 2, it was found that the obtained neonatal porcine bone marrow-derived mesenchymal stem cells (npBM-MSC) have a smaller average diameter than the human bone marrow-derived mesenchymal stem cells.

### [Evaluation of Growth Rate]

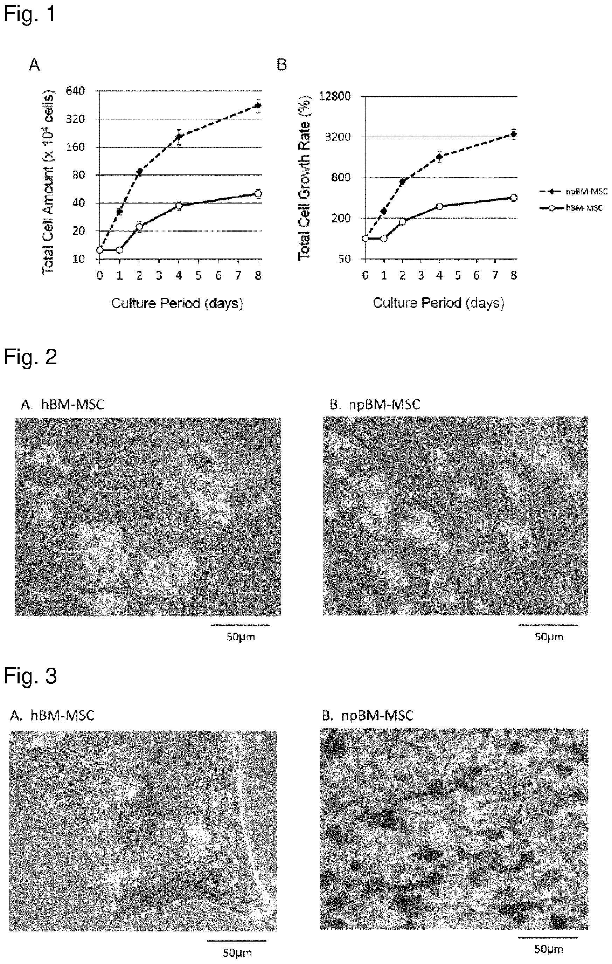

With respect to the human bone marrow-derived mesenchymal stem cells (hBM-MSC) and the neonatal porcine bone marrow-derived mesenchymal stem cells (npBM-MSC), the cells were inoculated into a T25 flask at a density of 5000 cells/cm² (1.25 × 10⁵ cells/flask) and cultured using the MSC medium. The MSC medium was replaced with a fresh one every three days. After 1, 2, 4, and 8 days from the start of culture, the total numbers of viable cells and dead cells were counted. The results are shown in Table 3 and Table 4, and also Fig. 1A and Fig. 1B. Note that the values in Table 3 and Table 4 are each the average ± SD (n=4).

**[Table 3]**

| Total cell amount (×10⁴ cells) | | | | | |
|---|---|---|---|---|---|
| Group | Immediately after suspension | Day 1 | Day 2 | Day 4 | Day 8 |
| npBM-MSC | 12.7 | 32.5 ± 2.7 | 86.9 ± 7.7 | 207.9 ± 38.2 | 448.0 ± 73.6 |
| hBM-MSC | 12.5 | 12.5 ± 0.8 | 22.3 ± 2.9 | 37.4 ± 3.8 | 50.5 ± 5.7 |

**[Table 4]**

| Total cell growth rate (%) | | | | | |
|---|---|---|---|---|---|
| Group | Immediately after suspension | Day 1 | Day 2 | Day 4 | Day 8 |
| npBM-MSC | 100 | 257 ± 22 | 687 ± 61 | 1643 ± 302 | 3542 ± 581 |
| hBM-MSC | 100 | 100 ± 7 | 178 ± 23 | 299 ± 31 | 404 ± 46 |

As shown in Table 3 and Table 4, and also in Fig. 1A and Fig. 1B, it was found that the obtained neonatal porcine bone marrow-derived mesenchymal stem cells (npBM-MSC) have a significantly higher growth rate than the human bone marrow-derived mesenchymal stem cells.

### [Differentiation into Adipocytes]

With respect to the human bone marrow-derived mesenchymal stem cells (hBM-MSC) and the neonatal porcine bone marrow-derived mesenchymal stem cells (npBM-MSC), differentiation into adipocytes was induced using hMSC differentiation BulletKit (trademark)-adipogeni, PT-3004 (manufactured by Lonza Walkersville, Inc.) according to the protocol. The results of staining using Oil Red manufactured by Sigma-Aldorich Co. LLC on day 17 after the start of induction are shown in Fig. 2A and Fig. 2B, respectively.

As shown in Fig. 2A and Fig. 2B, it was found that the obtained neonatal porcine bone marrow-derived mesenchymal stem cells (npBM-MSC) can differentiate into adipocytes in the same manner as the human bone marrow-derived mesenchymal stem cells.

### [Differentiation into Osteocytes]

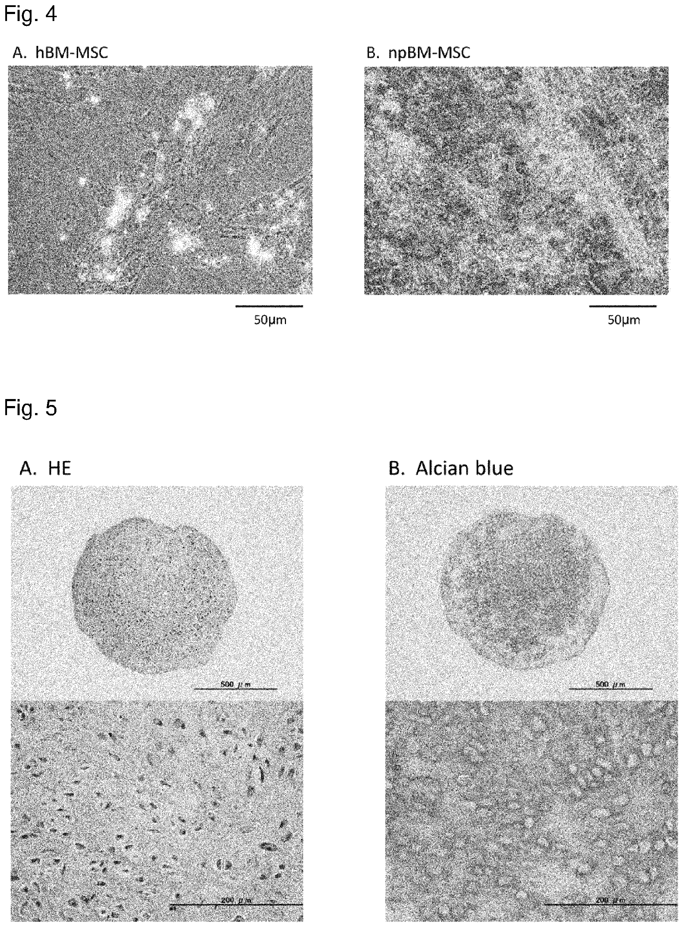

With respect to the human bone marrow-derived mesenchymal stem cells (hBM-MSC) and the neonatal porcine bone marrow-derived mesenchymal stem cells (npBM-MSC), differentiation into osteocytes was induced using hMSC differentiation BulletKit (trademark)-osteogenic, PT-3002 (manufactured by Lonza Walkersville, Inc.) according to the protocol. The results of staining using an alkaline phosphatase staining kit manufactured by Cosmo Bio Co., Ltd. on day 14 after the start of induction are shown in Fig. 3A and Fig. 3B, respectively, and the results of staining using a calcification staining kit manufactured by Cosmo Bio Co., Ltd. are shown in Fig. 4A and Fig. 4B, respectively.

As shown in Fig. 3A and Fig. 3B, and also in Fig. 4A and Fig. 4B, it was found that the obtained neonatal porcine bone marrow-derived mesenchymal stem cells (npBM-MSC) can differentiate into osteocytes in the same manner as the human bone marrow-derived mesenchymal stem cells.

### [Differentiation into Chondrocytes]

With respect to the neonatal porcine bone marrow-derived mesenchymal stem cells (npBM-MSC), differentiation into osteocytes was induced using hMSC differentiation BulletKit (trademark)-chondrogenic, PT-3003 (manufactured by Lonza Walkersville, Inc.) according to the protocol. The results of HE staining and the results of Alcian blue staining on day 19 after the start of induction are shown in Fig. 5A and Fig. 5B, respectively.

As shown in Fig. 5A and Fig. 5B, it was found that the obtained neonatal porcine bone marrow-derived mesenchymal stem cells (npBM-MSC) can differentiate into chondrocytes.

### Test Example 2

### [Culture of Cells in Neonatal Pig-Derived Monocytic Cell (npMNC) Fraction and Preparation of Neonatal Porcine Bone Marrow-Derived Mesenchymal Stem Cells (npBM-MSC)]

The MSC minimal essential medium or the MSC medium was left to stand for 10 to 15 minutes in an incubator (37°C, 5% CO₂) before use. In the same manner as in Test Example 1, the cell suspension containing the cells in the neonatal pig-derived monocytic cell (npMNC) fraction cryopreserved in a cryovial was promptly thawed in a water bath at 37°C. The thawed cell suspension was calmly added to 30 mL of the MSC minimal essential medium with temperature-equilibrated to 37°C, and the resultant was dispensed into two 50-mL tubes in an amount of 15 mL each.

Centrifugation was performed at room temperature for 5 minutes at 500×g, and the resulting pellet was resuspended in 2 mL of the temperature-equilibrated MSC minimal essential medium or MSC medium, and the suspension was gently pipetted up and down. The results of measurement of the total number of cells and the number of viable cells are shown below.
2 mL of the MSC minimal essential medium: the total number of cells: 2.60×10⁶, the number of viable cells: 4.8×10⁵, and the viability: 18.5%
2 mL of the MSC medium: the total number of cells: 2.55×10, the number of viable cells: 4.5×10⁵, and the viability: 17.6%

The cell suspension in an amount calculated so that the number of inoculated cells is as described below was added to a 6-well plate (without gelatin coat) in which the following medium was placed in each well, and the cell suspension was dispersed on a growth surface by gently shaking.
2 mL of the MSC minimal essential medium: the cells were inoculated at 2.60×10⁶ cells/well
2 mL of the MSC medium: the cells were inoculated at 2.55×10⁶ cells/well

The plate was placed in a CO₂ incubator, and incubated under the conditions of 37°C, 5% CO₂, and 90% humidity. After 3 days and after 6 days from the inoculation, the medium was replaced with the MSC medium to proliferate the cells, and on day 8 after the inoculation, the cells were subcultured.

### [Subculture]

After the neonatal porcine bone marrow-derived mesenchymal stem cells (npBM-MSC) reached nearly 50 to 60% confluence, the cells were recovered from one well and reinoculated into a T75 flask without gelatin coat.

The cells were washed with 2 mL of PBS (-), 320 µL of 0.25% trypsin was added per well, and the cells were left to stand for a few minutes in an incubator. After the cells were peeled off, the medium was neutralized with 1680 µL of the MSC medium. The cell suspension was collected in a 50-mL tube, and 8 mL of the MSC medium was added thereto, followed by centrifugation at room temperature for 5 minutes at 500×g.

To the obtained pellet, the temperature-equilibrated MSC medium (2 mL) was added, and the pellet was gently resuspended therein by pipetting up and down, and the results of measurement of the total number of cells and the number of viable cells are shown below.
a group of the MSC minimal essential medium at the time of inoculation of P0: the total number of cells: 5.0×10⁵, the number of viable cells: 5.0×10⁵, and the viability: 100%
a group of the MSC medium at the time of inoculation of P0: the total number of cells: 3.3×10⁵, the number of viable cells: 3.3×10⁵, and the viability: 100%

To a T75 flask (without gelatin coat), the MSC medium (15 mL) was added The neonatal porcine bone marrow-derived mesenchymal stem cells (npBM-MSC) were reinoculated into the flask so that the number of cells became as described below, and cultured in an incubator. The cells were defined as a first subculture.
a group of the MSC minimal essential medium at the time of inoculation of P0: the number of viable cells: 5.0×10⁵ cells/flask
a group of the MSC medium at the time of inoculation of P0: the number of viable cells: 3.3×10⁵ cells/flask

### [Preparation of Neonatal Porcine Bone Marrow-Derived Mesenchymal Stem Cells (npBM-MSC)]

After the reinoculated cells reached nearly 80 to 90% confluence according to the above-mentioned procedure, the cells were collected from one flask of the T75 flasks (without gelatin coat). The cells were washed with 8 mL of PBS (-), 2.4 mL of trypsin was added at 0.25 mL/well, and the cells were left to stand for a few minutes in an incubator. After the cells were peeled off, the medium was neutralized with 12.6 mL of the MSC medium. The cell suspension was collected in a 50-mL tube, followed by centrifugation at room temperature for 5 minutes at 500×g.

To the obtained pellet, the temperature-equilibrated MSC medium (5 mL) was added, and the pellet was calmly resuspended by pipetting up and down, and the results of measurement of the total number of cells and the number of viable cells are shown below. The cells from one flask (the MSC minimal essential medium for 3 days after the inoculation of PO): the total number of cells: 5.12×10⁶, the number of viable cells: 5.09×10⁶, and the viability: 99.5%

The cells from one flask (the MSC medium from the inoculation of PO): the total number of cells: 4.76×10⁶, the number of viable cells: 4.73×10⁶, and the viability: 99.4%

### [Cryopreservation of Neonatal Porcine Bone Marrow-Derived Mesenchymal Stem Cells (npBM-MSC)]

Apart from the above-mentioned culture, the early subculture cells were frozen in the same manner as in Test Example 1, whereby a cell stock was prepared.

### Test Example 3

A cell surface antigen on the neonatal pig-derived monocytic cells (npMNC) prepared in Test Example 1 and Test Example 2 was analyzed. The preparation method for each sample used in the analysis are shown in Table 5. In Table 5, "Switch" indicates that the culture was performed by using the MSC minimal essential medium (vitamin C-free) during initial culture, and changing the medium to the MSC medium (containing vitamin C) that is a growth medium during proliferation culture.

**[Table 5]**

| Preparation method for each sample | | | | |
|---|---|---|---|---|
| Sample No. | Medium | 0.1% gelatin coat | | Freezing condition |
| | | during initial culture | during proliferation culture | |
| 1 | Switch | with | with | CELLBANKER (registered trademark) |
| 2 | Switch | with | with | OPF-301 (10% DMSO) |
| 3 | Switch | with | without | CELLBANKER (registered trademark) |
| 4 | Switch | with | without | OPF-301 (10% DMSO) |
| 5 | Switch | without | without | OPF-301 (10% DMSO) |
| 6 | Only MSC medium | without | without | OPF-301 (10% DMSO) |

### [Analysis of Cell Surface Antigen]

Each cell sample was taken out from the liquid nitrogen tank, the cap was loosened to release the pressure, and the cap was closed again, and then, the sample was thawed in a thermostat bath preheated to 37°C while lightly stirring for 1 to 2 minutes. Each thawed cell was transferred to a 15-mL centrifuge tube containing 5 mL of Stain Buffer (manufactured by BD), followed by centrifugation at 4°C for 5 minutes at 500×g, and the supernatant was removed. Stain Buffer (5 mL) was added thereto, followed by centrifugation at 4°C for 5 minutes at 500×g, and washing was performed twice.

The cells were resuspended in 2 mL of Stain Buffer (manufactured by BD), and the number of viable cells was counted. Centrifugation was performed again (500×g, 5 minutes, 4°C), and the cells were resuspended in Stain Buffer (manufactured by BD) so that the cell density became 1×10⁷ cells/mL, and the cell suspension was dispensed into 1.5-mL tubes in an amount of 20 µL (cell count: 2×10⁵ cells) each, whereby four tubes in total were prepared for each of the following samples: unstained control, CD44, CD90, and Isotype Control.

To the corresponding tubes, 4 µL of Anti-CD44, Mouse (MEM-263), PE (manufactured by GeneTex, Inc.), 1 µL of PE Mouse Anti-Human CD90 (manufactured by BD) (having cross-reactivity with pig), and 4 µL of PE Mouse IgG1, κ Isotype Control (manufactured by BD) were added, followed by incubation for 45 minutes on ice under shading. The unstained control was also stored on ice.

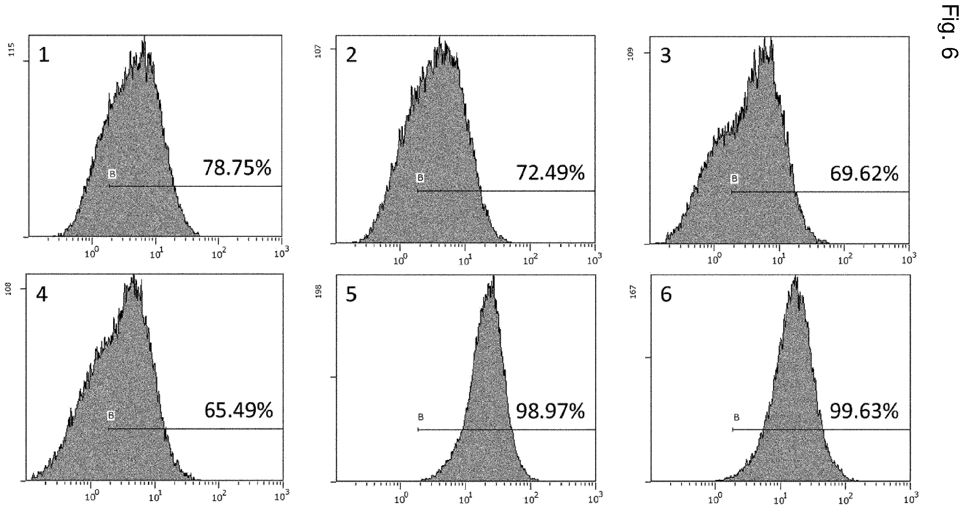

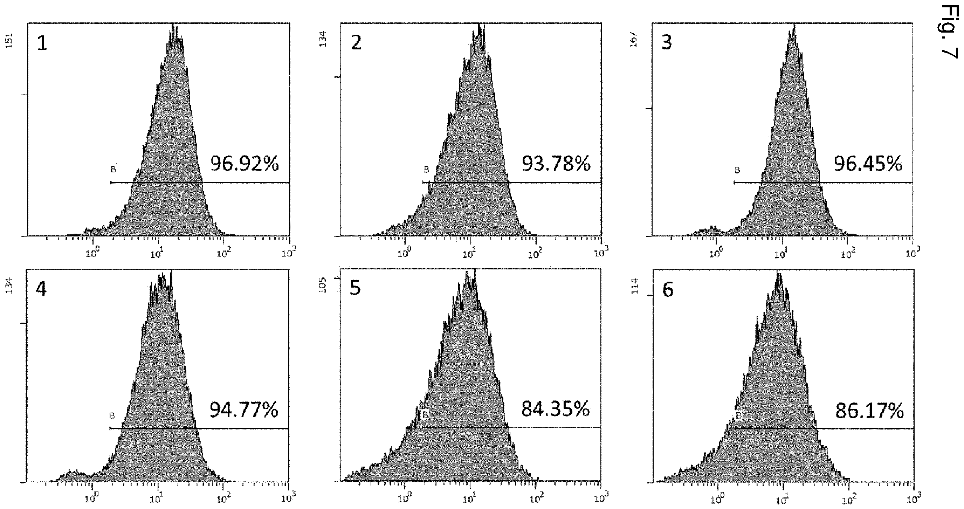

To each tube, 1 mL of Stain Buffer (manufactured by BD) was added, followed by centrifugation at 4°C for 5 minutes at 500×g, and washing was performed twice. The cell pellet was loosened by tapping and resuspended in 500 µL of Stain Buffer (manufactured by BD), and the suspension was passed through a filter immediately before the analysis and transferred to a test tube for flow cytometry. The sample was stored on ice under shading until the analysis, and analyzed using flow cytometry. The results of CD44 are shown in Fig. 6, and the results of CD90 are shown in Fig. 7.

As shown in Fig. 6 and Fig. 7, any sample was positive for CD44 and CD90 that are markers for mesenchymal stem cells. In addition, it is considered that objective mesenchymal stem cells could be established even without performing coating with gelatin during initial culture. Note that in any case, a nonspecific reaction was not observed in the measurement for the Isotype Control.

### Test Example 4

### [Preparation of Neonatal Porcine Pancreatic Islet-Derived Mesenchymal Stem Cells]

The pancreatic islet was collected from a neonatal pig, and was subjected to suspension culture, thereby preparing a cell cluster. The cell cluster was cryopreserved in the same manner as in Test Example 1. The neonatal porcine pancreatic islet cryopreserved in a cryovial was promptly thawed in a water bath at 37°C.

The thawed pancreatic islet suspension was calmly added to 30 mL of the MSC minimal essential medium adjusted to reach the temperature equilibrium (37°C) using a micropipette. The resultant was centrifuged at 4°C for 1 minute at 210×g. Note that in the case where the pancreatic islet was not frozen, after the pancreatic islet was precipitated at room temperature by free falling, the supernatant was removed. The resulting pellet was resuspended in 4 mL of the temperature-equilibrated MSC minimal essential medium, and the suspension was gently pipetted up and down.

To a 6-well plate, the pancreatic islet suspension was added, and the cell suspension was dispersed on a growth surface (without gelatin coat) by gently shaking. Inoculation was performed in 2 mL of the MSC minimal essential medium at the pancreatic islet/well in the range of 1650 IEQ to 2125 IEQ.

In a CO₂ incubator, culture was performed under the conditions of 37°C, 5% CO₂, and 90% humidity, and after 3 days, the medium was replaced with the MSC medium so as to proliferate the cells, and thereafter, the MSC medium was replaced with a fresh one every three days. The preparation conditions for the samples are shown in Table 6. After 6 days from the inoculation, 100% confluence was reached regardless of whether initial freezing was performed or not performed.

**[Table 6]**

| Sample No. | Origin | Conditions | Freezing at P0* | Subculture number at freezing* | Liquid amount (mL) | Cell density (×10⁶ total cells/mL) |
|---|---|---|---|---|---|---|
| 7 | pancreatic islet | On the day of preparing pancreatic islet (day 1) | without | P2 (OPF-301) | 1 | 2.23 |
| 8 | pancreatic islet | On day 3 after preparing pancreatic islet (day 4) | without | P2 (OPF-301) | 1 | 1.22 |
| 9 | pancreatic islet | On the day of preparing pancreatic islet (day 1) | with (OPF-301) | P2 (OPF-301) | 1 | 3.25 |
| 10 | pancreatic islet | On day 3 after preparing pancreatic islet (day 4) | with (OPF-301) | P2 (OPF-301) | 1 | 2.3 |

| | | | | | | |
|---|---|---|---|---|---|---|
| * The type of 10% DMSO is shown in parentheses. | | | | | | |

### [Subculture]

After the neonatal porcine pancreatic islet-derived mesenchymal stem cells (npISLET-MSC) reached about 80% to nearly 95%, the cells were recovered from two wells and reinoculated into a T75 flask without gelatin coat.

The cells were washed with 2 mL of PBS (not containing calcium and magnesium), 320 µL of 0.25% trypsin was added per well, and the cells were left to stand for a few minutes in an incubator. After the cells were peeled off, the medium was neutralized with 1680 µL of the MSC medium. The cell suspension was collected in a 50-mL tube using a 1-mL pipette, and 16 mL (8 mL × 2 wells) of the MSC medium was added thereto, followed by centrifugation at room temperature for 5 minutes at 500×g. The obtained pellet was gently resuspended in the temperature-equilibrated MSC medium (2 mL) using a pipette.

### [Average Diameter of Cells]

The MSC medium (20 mL) was added to T75 flasks without gelatin coat, and reinoculation was performed. The cells were cultured under the conditions of 37°C, 5% CO₂, and 90% humidity in a CO₂ incubator. The cells were defined as a first subculture. After 3 days from the inoculation of the first subculture, 100% confluence was reached regardless of whether initial freezing was performed or not performed. From this, it was found that the growth rate of mesenchymal stem cells prepared from the pancreatic islet of a neonatal pig is equivalent to that of mesenchymal stem cells prepared from the bone marrow of a neonatal pig. The average diameters of the obtained neonatal porcine pancreatic islet-derived mesenchymal stem cells are shown in Table 7.

**[Table 7]**

| Sample No. | 11 | 12 | 13 | 14 |
|---|---|---|---|---|
| Preparation conditions | without freezing immediately after preparing pancreatic islet | with freezing immediately after preparing pancreatic islet | without freezing on day 3 of culture after preparing pancreatic islet | with freezing on day 3 of culture after preparing pancreatic islet |
| Type of 10% DMSO solution at freezing | - | OPF-301 | - | OPF-301 |
| Average diameter | 12.1 µm | 12.2 µm | 12.0 µm | 12.3 µm |

As shown in Table 7, it was found that neonatal porcine pancreatic islet-derived mesenchymal stem cells can be prepared regardless of the freezing condition in the preparation of the pancreatic islet, and the average diameters are equivalent regardless of whether freezing is performed or not performed.

### [Analysis of Cell Surface Antigen]

Each cell sample was taken out from the liquid nitrogen tank, the cap was loosened to release the pressure, and the cap was closed again, and then, the sample was thawed in a thermostat bath preheated to 37°C while lightly stirring for 1 to 2 minutes. Each thawed cell was transferred to a 15-mL centrifuge tube containing 5 mL of Stain Buffer (manufactured by BD), followed by centrifugation at 4°C for 5 minutes at 500×g, and the supernatant was removed. Stain Buffer (5 mL) was added thereto, followed by centrifugation at 4°C for 5 minutes at 500×g, and washing was performed twice.

The cells were resuspended in 2 mL of Stain Buffer (manufactured by BD), and the number of viable cells was counted. Centrifugation was performed again (500×g, 5 minutes, 4°C), and the cells were resuspended in Stain Buffer (manufactured by BD) so that the cell density was 1×10⁷ cells/mL. The cell suspension was dispensed into 1.5-mL tubes in an amount of 20 µL (cell count: 2×10⁵ cells) each, whereby four tubes in total were prepared for each of the following samples: unstained control, CD29, CD44, and CD90.

To the corresponding tubes, 1 µL of Mouse Alexa Fluor 647 Mouse Anti-Pig CD29 (manufactured by BD), 4 µL of Anti-CD44, Mouse (MEM-263), PE (manufactured by GeneTex, Inc.), and 1 µL of PE Mouse Anti-Human CD90 (manufactured by BD) (having cross-reactivity with pig) were added, followed by incubation for 45 minutes on ice under shading. The unstained control was also stored on ice.

To each tube, 1 mL of Stain Buffer (manufactured by BD) was added, followed by centrifugation at 4°C for 5 minutes at 500×g, and washing was performed twice. The cell pellet was loosened by tapping and resuspended in 500 µL of Stain Buffer (manufactured by BD), and the suspension was passed through a filter immediately before the analysis and transferred to a test tube for flow cytometry. The sample was stored on ice under shading until the analysis, and analyzed using flow cytometry.

The results of CD29 are shown in Fig. 8A to Fig. 8D, the results of CD44 are shown in Fig. 9A to Fig. 9D, and the results of CD90 are shown in Fig. 10A to Fig. 10D. Fig. 8A, Fig. 9A, and Fig. 10A show the results of Sample 11 (without freezing immediately after preparation of the pancreatic islet); Fig. 8B, Fig. 9B, and Fig. 10 B show the results of Sample 12 (with freezing immediately after preparation of the pancreatic islet); Fig. 8C, Fig. 9C, and Fig. 10C show the results of Sample 13 (without freezing on day 3 of culture after preparation of the pancreatic islet); and Fig. 8D, Fig. 9D, and Fig. 10D show the results of Sample 14 (with freezing on day 3 of culture after preparation of the pancreatic islet).

As shown in Fig. 8A to Fig. 10D, in any sample, a high positive rate was observed for CD29, CD44, and CD90 that are markers for mesenchymal stem cells. In addition, it is considered that objective mesenchymal stem cells could be established regardless of whether freezing is performed or not performed at initial culture.

Although the invention has been described in detail with reference to specific embodiments, it will be apparent to those skilled in the art that various changes and modifications can be made without departing from the spirit and scope of the invention. This application is based on US Provisional Application (US 62/555,913) filed on September 8, 2017, the contents of which are incorporated herein by reference. In addition, all references cited herein are incorporated in their entirety.

## Claims

1. Stem cells isolated from a neonatal pig.

2. The stem cells according to claim 1, wherein an average diameter is 17 µm or less.

3. The stem cells according to claim 1 or 2, wherein a doubling time in a logarithmic growth phase is 36 hours or less.

4. The stem cells according to any one of claims 1 to 3, wherein the stem cells are mesenchymal stem cells.

5. The stem cells according to any one of claims 1 to 4, wherein the neonatal pig is a neonatal pig that enables cell transplantation into a human.

6. The stem cells according to any one of claims 1 to 5, wherein the stem cells are isolated from the bone marrow or the pancreatic islet of the neonatal pig.

7. The stem cells according to any one of claims 1 to 6, wherein the stem cells are stem cells for transplantation.

8. The stem cells according to claim 7, wherein the stem cells are stem cells for human transplantation.

9. The stem cells according to any one of claims 1 to 6, wherein the stem cells are stem cells for feeder cells.

10. The stem cells according to claim 9, wherein the stem cells are stem cells for feeder cells for proliferating stem cells for human transplantation.

11. A method for preparing stem cells, comprising a step of isolating cells from a neonatal pig.

12. The method for preparing stem cells according to claim 11, comprising a step of subculturing the stem cells after 3 to 12 days from inoculation.

13. The method for preparing stem cells according to claim 11 or 12, wherein the cells to be isolated from the neonatal pig are cells in a monocytic cell fraction.

14. The method for preparing stem cells according to any one of claims 11 to 13, wherein the isolation of the cells from the neonatal pig is isolation of the cells from the bone marrow or the pancreatic islet of the neonatal pig.

15. The method for preparing stem cells according to claim 13 or 14, comprising a step of freezing the isolated cells in the monocytic cell fraction.
